# EUROPEAN PATENT SPECIFICATION

(11) **EP 1 149 276 B1**
(45) Date of publication and mention of the grant of the patent: **15.06.2005**
(21) Application number: 00901354.1
(22) Date of filing: 04.01.2000
(51) Int. Cl.: G01N 3/08, G01N 33/34

(54) **METHOD OF DETERMINING MECHANICAL PROPERTIES OF A SHEET MATERIAL**
VERFAHREN ZUR BESTIMMUNG MECHANISCHER EIGENSCHAFTEN EINES DRUCKBOGENMATERIALS
PROCEDE DE DETERMINATION DES PROPRIETES MECANIQUES DU MATERIAU D'UNE FEUILLE

(30) Priority: 08.01.1999 SE 9900026
(43) Date of publication of application: 31.10.2001
(73) Proprietor: PAPERPROBE Sweden AB, 187 37 Täby (SE)
(72) Inventor: ANTTILA, Jorma, S-168 68 Bromma (SE)
(74) Representative: Onn, Thorsten
(86) International application number: PCT/SE2000/000009
(87) International publication number: WO 2000/040944

(56) References cited:
- GB-A- 2 153 533
- US-A- 4 448 079
- US-A- 5 575 078

## Description

The present invention relates to a method of determining mechanical properties in the thickness direction of a sheet material, an apparatus for carrying out such a method, and a use of a body and a sensor in an apparatus for determining mechanical properties of a sheet material.

In the production of a sheet material such as paper, great demands are made upon, among other things, printability as well as the mechanical properties of the paper. A large amount of parameters in the papermaking process influence the paper quality. For the purpose of controlling the production of paper, as well as improving the quality and strength of a paper, it is important to determine physical quantities of the paper in efficient, fast and accurate ways. The quality and strength of a paper are dependent on mechanical properties such as compressive modulus, tensile strength, etc. The quality is also dependent on physical quantities such as surface roughness. The surface roughness and consequently the thickness of a paper sheet may vary across the paper web and as a function of time. These variations influence, among other things, the printability.

By the known methods, accurate mechanical properties as well as actual physical characteristics of a paper material are fairly complicated to determine. In the past, compressive modulus in the thickness direction has been difficult to determine at small deformations (pressures), and for single sheets of low grammage. Even a quantity such as thickness, which for many reasons is important to determine, is not quite easy to measure. In a commonly used standard method, the thickness is determined by placing weights on a piece of paper having a specific cut-out size. After a certain time the thickness of the paper is measured. In the known methods for determining compressive modulus in the thickness direction, the paper material is often ruptured or in other way destroyed, and can thereafter not be used for other tests or the purpose it is intended for. Further, a satisfactory method for determining compressive modulus of a thin paper material, e.g. newsprint paper, is not known in the art.

During the last decades, several methods have been proposed for determining compressive modulus in the thickness direction of a paper material. However, all of the proposed methods for determining the static or dynamic compressive modulus, have in common that at least two variables must be measured. For example, to determine the static compressive modulus, both the thickness and the pressure used must be registered. Accordingly, the change over time in both the thickness and pressure has to be measured for the dynamic compressive modulus.

Appita Journal, Vol.50, May 1997, No.3, Ting, T.H.D. et al, "Network changes in paper under compression in z-direction: the effect of loading rate and fibre wall thickness", p.223-229, discloses results from a method of measuring mechanical properties of paper such as compressibility. Standardised samples of handsheets were cut and dried, fitted and aligned in a testing chamber where three different loading rates were used.

Löytty, H. et al, "Statistical approach to paper compressibility", Progress in paper physics (1998), Vancouver, Canada, Aug. 9-14, p.1-6, relates to a statistical approach to determine paper compressibility, wherein a mathematical model and experiments are used to study paper compressibility.

US-A-5,575,078 discloses a measurement apparatus and a method for measuring the thickness of sheets of material. A second contact face and plunger arm is advanced by a moving motor towards a first contact face, upon which a sheet or other workpiece rests. The first contact face, or anvil, includes a load cell which responds to the weight or force exerted on the anvil. In addition, the second contact face is connected to a position indicating system which produces an output representative of the position of the plunger. A microprocessor continuously receives and monitors the load and position signals. However, the necessity of measuring the weight/pressure on the sheet, a motor for advancing the second contact face and to have a computer/microprocessor for correlation of several signals, makes the apparatus and method relatively complicated and time-consuming.

GB 2 153 533 discloses equipment for measuring of the thickness and the compressibility of paper, comprising a supporting surface for the sheet material, a measuring wheel exercising pressure on the paper and means for measuring the movement of a measuring head and a compressive force. With this equipment, two parameters, namely the movement of a measuring head and the compressive force are being measured, and the equipment consists of different kinds of measuring heads for measuring different characteristics of the paper. This deteriorates the accuracy of the determination of the properties.

The purpose of the present invention is to provide a method which is uncomplicated and efficient for determining mechanical properties and physical quantities/characteristics in the z-direction of a sheet material. An object is to provide a method for determining mechanical properties and physical quantities on surfaces of varying size, i.e. within a wide range in spatial resolution (at least in the range of about 1-1000 mm²). It is also a purpose to provide a method which is accurate and fast. A further object is to provide a method for determining mechanical properties and physical quantities in the z-direction while using relatively small deformations (pressures). Yet a further purpose is to provide a method where the sheet material can be subjected to the method without rupturing or in other ways destroying the sheet, alternatively causing the smallest possible influence on the sheet material.

The present invention provides a method of determining mechanical properties in the thickness direction of a sheet material, by supporting the sheet material on a surface; advancing a body having an impacting surface facing the sheet material into engagement with the sheet material supported by said surface; sensing and registering one parameter, said parameter being either the position, velocity or acceleration of the impacting surface of the advancing body as a function of time during the movement of the body in relation to a sensor, so that said function includes only one sensed parameter; and analysing said function to obtain the mechanical properties.

An advantage of the method of the present invention is that mechanical properties are possible to determine solely by measuring only one parameter over time, namely the position, velocity or acceleration of the impacting surface of the body, to determine several mechanical properties in the thickness direction. With the present method, it is also possible to determine the mechanical properties and physical quantities by the use of a relatively small deformation (pressures), without rupturing or in other ways destroying the sheet and causing a minimum of influence on the sheet material. Hence, with the method of the present invention it is even possible to determine the mechanical properties of a thin quality of a paper material, e.g. a newsprint paper. In addition, the method is fast, uncomplicated, accurate and efficient.

The present invention also provides for an apparatus for determining mechanical properties in the thickness direction of a sheet material, the apparatus comprises a supporting surface for the sheet material, an advancing body having an impacting surface facing the sheet material, the body is attached to a supporting structure, where said body is movably arranged for engagement with the sheet against said surface, a sensor is arranged for sensing and registering one parameter, said parameter being either the position, velocity or acceleration of the impacting surface of the advancing body, as a function of time during the movement of the body in relation to the sensor so that said function includes only one sensed parameter, and analysing said function to obtain the mechanical properties.

The method can be used to measure mechanical properties on different kinds of sheet materials, i.e. paper, plastics, cork, foamed solids, etc. These are few examples of non-metal materials that all have a compressive modulus in the thickness direction that is less than the compressive modulus of the supporting surface that holds the sample when measured. The less the compressive modulus in the sheet is, in relation to the material of the supporting surface, the better. As an example, paper has a compressive modulus that is more than four orders of magnitude less than aluminium or silica glass. It is also possible to easily account and compensate for the known compressive modulus of the material of the supporting surface when the position, alternatively the velocity or acceleration, over time of the impacting surface of the body is analysed to obtain the mechanical properties of the sheet. Where the expressions "paper" or "paper material" are used in the description, there is also meant a web of paper or a web of paperboard. The paper material can be made of a lignocellulose-containing pulp comprising recirculated fibres, fresh fibres or a mixture thereof. It may also contain added chemicals and filler materials, or have been subjected to various surface treatments, or covered with some kind of coating layers. Consequently, the expression "paper" may also include thinner and lighter paper qualities, such as newsprint paper.

With the method according to the present invention, it can be possible to determine mechanical properties in the thickness direction of a sheet material having a thickness as thin as about 50 µm.

Mechanical properties such as dynamic compression modulus, elastic and viscoelastic behaviour, etc., can be determined with the method of the present invention. In addition, surface compressability and thickness of a sheet material can be determined with the method.

The sheet material intended to be tested in the method, is suitably supported on a flat surface which can be substantially horizontally arranged. The supporting surface includes means known in the art, which makes it possible to hold a sheet in a plain fixed position, e.g. by suction with air.

According to the present invention, a sensor is arranged for sensing and registering the position, velocity or acceleration of the impacting surface of the advancing body. The sensor is arranged in the vicinity of the body and suitably at the supporting surface. However, it can be possible to arrange the sensor for instance in the supporting structure, above the body. The sensor is preferably placed on the opposite side of the sheet material in relation to the advancing body. The sensor registers and senses the position, alternatively the velocity or acceleration, of the impacting surface of the advancing body as a function of time during the movement of the body towards the sheet material. Both capacitive and inductive displacement sensors have the needed resolution (nanometers) and accuracy (about 0.1 % full scale) to be used in the present invention. The preferred inductive (eddy current) sensor is not sensitive to dirt, grease or other contamination that could be present, and neither to environmental changes such as water (humidity). The capacitive displacement sensor might need a grounded target, which complicates the design of the moving body.

As mentioned above, in the method according to the present invention, the sensor registers and senses the position, alternatively the velocity or acceleration, of the impacting surface of the advancing body as a function of time. In the following is described how mechanical properties are determined when said function is analysed, if sensing and registering the position of the impacting surface of the advancing body: A body having a mass, m, is released from a height, h, against a sheet placed on a supporting surface. The position of the impacting surface of the advancing body is sensed and registered, as a function of time, s(t), during the movement of the body in relation to the supporting surface of the sheet material. When the impacting surface of the body comes into contact with the sheet, the retardation of the body starts. When this occurs, the acceleration, **a(t),** differs from the acceleration of gravity, **g. A(t)** is determined by differentiating **s(t)** two times with respect to time. At this point in time **t**_{**0**}**,** the thickness, **s**_{**0**}**,** can be determined from **s**_{**0**}**=s(t**_{**0**}**).** The maximum level of strain, **ε(t),** can be varied by choosing the mass, m, of the body and the height, **h,** from which the body is released. The retardation force, **F(t),** is determined by differentiating **s(t)** two times with respect to time, subtract the acceleration of gravity, **g,** and multiply with the mass, **m** (Newton's second law of motion). Simultaneously, the linear or logarithmic strain, **ε**, is obtained from **s**_{**0**}**- s(t)** divided by **s**_{**0**}**.** Consequently, the mechanical properties can be determined from the diagram **F(t)** vs. **ε(t).**

According to an embodiment of the method of the present invention, the sensing and registering of the position, velocity or acceleration of the impacting surface of the body is carried out during retardation until of the body is at rest as well. Thus, the sensing and registering can be followed until static loading, depending on how much and which information that is desired (compression modulus, elastic and viscoelastic behaviour, etc.). In order to determine a specific mechanical property, a certain part of the function in the diagram is studied.

The present invention also provides for a use of a body and a sensor in a method or an apparatus according to the present invention, for determining mechanical properties of a sheet material.

The body can be of a suitable material, having preferably a longitudinal elongation with the geometrical shape of a rod, piston, bar, mast, pole, cylinder or the similar. The body may have any cross sectional area. The body is preferably a metal rod with a substantially circular cross section. It is also possible that the body comprise of several body-weights that can be removed or added, such as a threaded sleeve. In this case the mass of the body can easily be altered. The body is suitably arranged so as to be tightly constrained in lateral direction, transverse its longitudinal extension, but still movable with negligible friction losses in its longitudinal extension towards a sheet surface. The orientation of the apparatus for carrying out the method according to the present invention relative to the direction of the gravitational force, is not of any major importance. But it is convenient to let the force of gravity advance the body towards the sheet surface. But it is possible to use other forces to accelerate the body to frictionless advancement towards the sheet surface and thus change the orientation of the whole method and apparatus, but the principle of the present invention would still be the same. Hence, the end of the body facing the sheet surface, herein disclosed as the impacting surface, is preferably arranged horizontally. The rear end surface of the body is suitably smooth and even. Said end surface of the body is suitably arranged parallel to the sheet surface.

The body is attached to a supporting structure, where said body is movably arranged, with generally zero friction. The supporting structure as well as the mechanism for releasing the body can be of a conventional construction, well known for the person skilled in the art. The material in the supporting structure is chosen to minimise distortion caused by thermal expansion.

The sensor used for sensing and registering the position, velocity or acceleration of the impacting surface of the advancing and retarding body can be of a kind generally available on the market. Preferably, an inductive (eddy current) displacement sensor is used in the present method and apparatus according to the invention. The sensor should be able to sense and register at parts of millimetres. Suitably the sensor can be able to sense and register nanometers. The sensing and registration by the sensor is preferably carried out continuously or digitised with a sufficient sampling rate during the advancing movement of the body against the sheet material, as well as during the retardation of the body. Although only one sensor, providing only one output signal, is needed according to the present invention, it is within the scope of the invention that in addition to said above mentioned sensor use several other sensors for measuring different variables. However, according to the preferred embodiment of the present invention, only one single sensor is used.

With reference to the accompanying drawings, embodiments of the present invention are described, without restricting the scope of the present invention thereto.

Fig. 1 is a schematic side view of an apparatus for carrying out the method herein disclosed.

Fig. 2a is an enlarged side view of a body positioned at a distance from a sheet and a supporting surface, while fig. 2b shows an enlarged side view of an alternative embodiment of the body.

Fig. 3 illustrates in a principle presentation in diagrams the position s of an impacting surface, the velocity and the acceleration respectively, as a function of time.

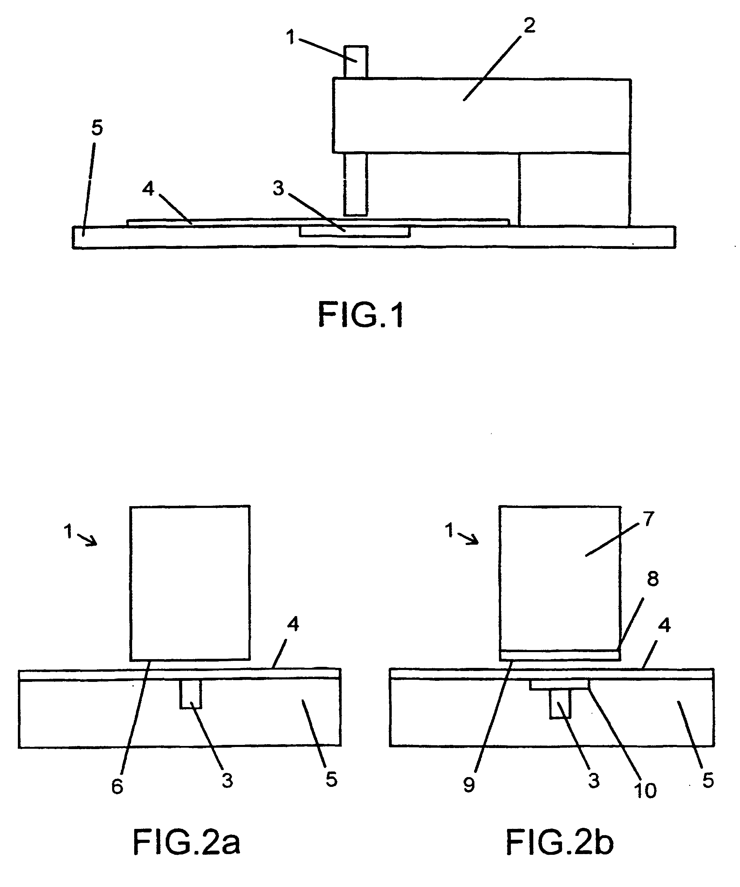

As evident from fig. 1, the present invention provides an apparatus for determining mechanical properties of a sheet material 4. The apparatus comprises a supporting surface 5 for the sheet material 4. A body 1 is attached to and laterally constrained by a supporting structure 2. The impacting surface of the body 1 is movably arranged for engagement with the sheet. A sensor 3 is placed in the supporting surface 5, thus being a supporting surface itself, more particularly arranged on the opposite side of the sheet material 4 for sensing and registering the position, alternatively the velocity or acceleration, of the impacting surface of the advancing body 1.

Hence, according to the method of the present invention, a body 1 is released from a height above a sheet material against the sheet 4, which is placed on a supporting surface 5. The position, velocity or acceleration of the impacting surface of the advancing body is sensed and registered by the sensor 3, as a function of time, during the movement of the body in relation to the sensor 3. Subsequently, when the position, alternatively the velocity or acceleration, of the impacting surface of the advancing body has been registered as a function of time, said function is analysed to obtain the desired mechanical properties.

Fig. 2a illustrates an enlarged view of a body 1 positioned at a distance from a sheet 4 and a supporting surface 5. The body 1 has an impacting surface 6 facing the sheet material 4. The body 1 including the impacting surface can be made of aluminium in one piece as shown in fig. 2a or in two or more pieces. Fig. 2b illustrates the body 1 divided in two parts, a first body member 7 and a second body member 8, which is facing the sheet material 4. The first body member can be e.g. a piston and the second body member can be called a target. The material and design of the piston 7 are optimised for the frictionless and laterally constrained movement, in direction normal to the measured sheet 4. The target 8 can be a thin member, e.g. a thin disc, of a second material at the end of the piston 7 that is facing the measured sheet 4. This target has a material thickness and a design optimised to the needs of the sensor 3 that measures the position, velocity or acceleration. The diameter of the target could be the same or different from the piston to enable an easy way of changing the spatial resolution. The target material is preferably, due to the inductive (eddy current) sensor, made of a non-magnetic material, i.e. aluminium, brass, copper, etc. The impacting surface 9 of the target 8 could, if necessary for the purpose of producing a more uniform stress field, be provided with a suitable thin layer of soft material (e.g. neoprene rubber) that conforms to the irregularities of the sheet material, e.g. if measuring on a paper. With the introduction of this layer, the influence of surface roughness can be suppressed. The sensor 3 can be placed integrated with the supporting surface 5 so that the sensor surface coincides with the surface of the supporting structure 5. Alternatively as shown in fig. 2b, a thin additional supporting surface 10 with known physical properties and dimensions is placed above the displacement sensor 3. This additional supporting surface 10 serves only as protection of the displacement sensor 3. The influence of this known material and also the suitably used thin layer of soft material (normally it will not have any significant effect on the accuracy) is easily accounted and compensated for when the position, velocity or acceleration over time of the impacting surface of the body 1 is analysed to obtain the mechanical properties of the sheet.

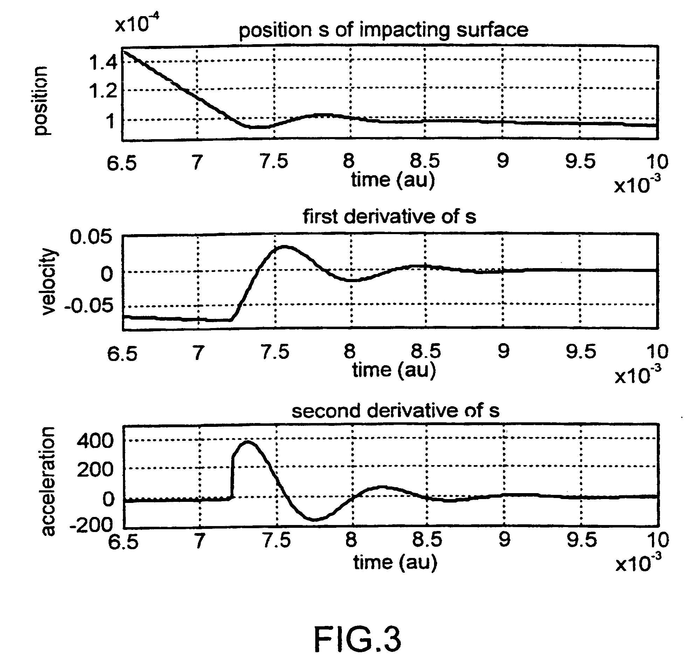

As evident from fig. 3, a principle presentation shows in diagrams the position s of an impacting surface, the velocity and the acceleration respectively, as a function of time **s(t)**. In the examples illustrated in the diagrams, the y-axis represents position, velocity and acceleration respectively, in µm. The x-axis represents the time in milliseconds. However, other multiple of the units can be used, such as meter and seconds. For the purpose of illustrating the principle of the analysis according to the present invention, a determination can be made as follows: From the principle diagrams in fig. 3, the thickness, so, can be determined from **s**_{**0**}**=s(t**_{**0**}**).** As evident from the diagram showing the acceleration as a function of time, **t**_{**0**} is at about 7,2 milliseconds on the x-axis. At this point in time **t**_{**0**}**,** the thickness, **S**_{**0**}**,** can easily be determined from the uppermost diagram, showing the position s as a function of time **s(t).** Consequently, at 7,2 milliseconds, the thickness (position) **s**_{**0**} is about 1 µm.

## Claims

1. Method of determining mechanical properties in the thickness direction of a sheet material (4), comprising the steps of supporting the sheet material (4) on a surface (5) and advancing a body (1) having an impacting surface (6, 9) facing the sheet material into engagement with the sheet material (4) supported by said surface (5), **characterised by**
sensing and registering one parameter, said parameter being either the position, velocity or acceleration of the impacting surface (6, 9) of the advancing body (1) as a function of time during the movement of the body (1) in relation to a sensor (3), so that said function includes only one sensed parameter; and
analysing said function to obtain the mechanical properties.

2. The method according to claim 1, **characterised in that** the position, velocity or acceleration of the impacting surface (6, 9) of the body (1) is continuously registered by an inductive displacement sensor (3).

3. The method according to claim 1 or 2, **characterised in that** the mechanical property determined is compressive modulus.

4. The method according to any of the preceding claims, **characterised in that** the sensing and registering of the position, velocity or acceleration of the body (1) is carried out during retardation until the body (1) is at rest as well.

5. The method according to claim 1, **characterised in that** the body (1) is movable in a vertical direction.

6. Apparatus for determining mechanical properties in the thickness direction of a sheet material (4), comprising a supporting surface (5) for the sheet material (4), and an advancing body (1) having an impacting surface (6, 9) facing the sheet material, the body is attached to a supporting structure (2) where said body (1) is movably arranged for engagement with the sheet, **characterised in that** the apparatus comprises, a sensor (3) arranged for sensing and registering one parameter said parameter being either the position, velocity or acceleration of the impacting surface (6, 9) of the advancing body (1), as a function of time during the movement of the body (1) in relation to the sensor (3) so that said function includes only one sensed parameter, and analysing said function to obtain the mechanical properties.

7. Apparatus according to claim 6, **characterised in that** sensor (3) is arranged at the supporting surface (5), said sensor (3) is arranged on the opposite side in relation to the advancing body (1).

8. Apparatus according to claim 6 or 7, **characterised in that** the body (1) is a rod with a substantially circular cross section.

9. Apparatus according to claim 6-8 , **characterised in that** the body is divided in two parts, a first body member (7) and a second body member (8).

10. Apparatus according to claim 6, **characterised in that** said sensor is an inductive displacement sensor (3).

11. Use of a body (1) and a sensor (3) in a method according to claims 1-5 or in an apparatus according to claims 6-10, for determining mechanical properties of a sheet material (4).

## Patentansprüche

1. Verfahren zum Bestimmen der mechanischen Eigenschaften von blattartigern Material (4) in Richtung seiner Dicke, bei welchem das blattartige Material (4) auf eine Unterlage (5) gelegt wird und bei welchem ein Körper (1) mit einer in Richtung des blattartigen Materials (4) weisenden Anlagefläche (6,9) bis zur Anlage am blattartigen Material (4) gebracht wird, das von der Unterlage (5) getragen wird,
**dadurch gekennzeichnet,**
- **daß** in Abhängigkeit von der Zeit, in welcher der Körper (1) relativ zu einem Sensor (3) bewegt wird, ein Parameter gemessen und erfaßt wird, welcher entweder die Position, die Geschwindigkeit oder die Beschleunigung der Anlagefläche (6,9) des Körpers (1) ist, so daß im Ergebnis nur ein Parameter gemessen wird, und
- **daß** das Ergebnis der Messung zur Bestimmung der mechanischen Eigenschaften analysiert wird.

2. Verfahren nach Anspruch 1, **dadurch gekennzeichnet, daß** Position, Geschwindigkeit oder Beschleunigung der Anlagefläche (6,9) des Körpers (1) kontinuierlich durch einen induktiven Verschiebesensor (3) gemessen wird.

3. Verfahren nach Anspruch 1 oder 2, **dadurch gekennzeichnet, daß** die gemessene mechanische Eigenschaft ein Modul der Zusammendrückbarkeit ist.

4. Verfahren nach einem der Ansprüche 1 bis 3, **dadurch gekennzeichnet, daß** die Messung und die Erfassung von Lage, Geschwindigkeit oder Beschleunigung des Körpers (1) auch während der Verlangsamung des Körpers (1) bis zu seinem Stillstand durchgeführt wird.

5. Verfahren nach Anspruch 1, **dadurch gekennzeichnet, daß** der Körper (1) vertikal bewegbar ist.

6. Vorrichtung zum Bestimmen der mechanischen Eigenschaften von blattartigern Material (4) in Richtung seiner Dicke, welche eine Unterlage (5) für das blattartige Material (4) und einen verschiebbaren Körper (1) mit einer in Richtung des blattartigen Materials (4) weisenden Anlagefläche (6,9) aufweist, der an einem Träger (2) bewegbar angebracht ist, um zur Anlage am blattartigen Material (4) gebracht zu werden, **dadurch gekennzeichnet, daß** in der Vorrichtung ein Sensor (3) zum Bestimmen und Erfassen eines Parameters in Abhängigkeit von der Zeit, in welcher der Körper (1) relativ zum Sensor (3) bewegt wird, vorhanden ist, welcher Parameter entweder die Position, die Geschwindigkeit oder die Beschleunigung der Anlagefläche (ö,9) des verschiebbaren Körpers (II) isf, so daß im Ergebnis nur ein Parameter gemessen und das Ergebnis der Messung zur Bestimmung der mechanischen Eigenschaften analysiert wird.

7. Vorrichtung nach Anspruch 6, **dadurch gekennzeichnet, daß** der Sensor (3) an der Unterlage (5) angebracht ist, auf einer dem zu bewegenden Körper (1) gegenüber liegenden Seite.

8. Vorrichtung nach Anspruch 6 oder 7, **dadurch gekennzeichnet, daß** der Körper (1) eine Stange mit im wesentlichen kreisförmigem Querschnitt ist.

9. Vorrichtung nach einem der Ansprüche b bis 8, **dadurch gekennzeichnet, daß** der Körper (1) in zwei Teile geteilt ist, einen ersten Abschnitt (7) und einen zweiten Abschnitt (8).

10. Vorrichtung nach Anspruch 6, **dadurch gekennzeichnet, daß** der Sensor ein induktiver Verschiebesensor (3) ist.

11. Verwendung von einem Körper (1) oder Sensor (3) in ein Verfahren nach einem der Ansprüche 1 bis 5 oder in eine Vorrichtung nach einem der Ansprüche 6 bis 10, zum Bestimmen der mechanischen Eigenschaften von blattartigem Material.

## Revendications

1. Un procédé de détermination des propriétés mécaniques dans la direction de l'épaisseur du matériau (4) d'une feuille, comprenant les étapes consistant à supporter le matériau (4) de la feuille sur une surface (5) et à faire avancer un corps (1) présentant une surface d'impact (6, 9) dirigée vers le matériau de la feuille en engagement avec le matériau (4) de la feuille supporté par ladite surface (5),
**caractérisé par** les étapes consistant à
détecter et enregistrer un paramètre, ledit paramètre étant soit la position, soit la vitesse, soit l'accélération de la surface d'impact (6, 9) du corps avançant (1) en fonction du temps pendant le déplacement du corps (1) par rapport à un capteur (3), de sorte que ladite fonction ne comprend qu'un seul paramètre détecté ; et
analyser ladite fonction pour obtenir les propriétés mécaniques.

2. Le procédé selon la revendication 1, **caractérisé en ce que** la position, la vitesse ou l'accélération de la surface d'impact (6, 9) du corps (1) est enregistrée de manière continue par un capteur de déplacement inductif (3).

3. Le procédé selon la revendication 1 ou 2, **caractérisé en ce que** la propriété mécanique déterminée est un module de compression.

4. Le procédé selon l'une quelconque des revendications précédentes, **caractérisé en ce que** l'étape consistant à détecter et à enregistrer la position, la vitesse ou l'accélération du corps (1) est mise en oeuvre pendant un ralentissement jusqu'à ce que le corps (1) soit lui-même au repos.

5. Le procédé selon la revendication 1, **caractérisé en ce que** le corps 1 est déplaçable dans une direction verticale.

6. Appareil pour déterminer des propriétés mécaniques dans la direction de l'épaisseur du matériau (4) d'une feuille, comprenant une surface de support (5) pour le matériau (4) de la feuille et un corps (1) qui avance en présentant une surface d'impact (6, 9) dirigée vers le matériau de la feuille, le corps est fixé à une structure de support (2) où ledit corps (1) est disposé de manière mobile pour un engagement avec la feuille, **caractérisé en ce que** l'appareil comprend un capteur (3) disposé pour détecter et enregistrer un paramètre, ledit paramètre étant soit la position, soit la vitesse, soit l'accélération de la surface d'impact (6, 9) du corps avançant (1) en fonction du temps pendant le déplacement du corps (1) par rapport au capteur (3) de sorte que ladite fonction ne comprend qu'un seul paramètre détecté, et analyser ladite fonction pour obtenir les propriétés mécaniques.

7. Appareil selon la revendication 6, **caractérisé en ce que** le capteur (3) est disposé au niveau de la surface de support (5), ledit capteur (3) étant disposé sur le côté opposé par rapport au corps (1) qui avance.

8. Appareil selon la revendication 6 ou 7, **caractérisé en ce que** le corps (1) est une tige à section transversale sensiblement circulaire.

9. Appareil selon la revendication 6-8, **caractérisé en ce que** le corps est divisé en deux parties, un premier élément de corps (7) et un second élément de corps (8).

10. Appareil selon la revendication 6, **caractérisé en ce que** ledit capteur est un capteur de déplacement inductif (3).

11. Utilisation d'un corps (1) et un capteur (3) dans un procédé selon la revendication 1-5 ou dans un appareil selon la revendication 6-10, pour détermination des propriétés mécaniques du matériau (4) d'une feuille.
